# EUROPEAN PATENT SPECIFICATION

(11) **EP 1 433 434 B1**
(45) Date of publication and mention of the grant of the patent: **02.11.2005**
(21) Application number: 02028934.4
(22) Date of filing: 23.12.2002
(51) Int. Cl.: A61C 1/00, A61L 2/18

(54) **A method for sterilising/disinfecting the water circuit of a dental unit and a dental unit implementing the method**
Verfahren zur Sterilisation/Disinfektion des Wasserkreislaufs einer zahnärtzlichen Einheit und eine dieses durchführende zahnärtliche Einheit
Méthode pour stériliser/désinfecter le circuit d'eau d'une unité pour soins dentaires et unité pour soins dentaires la mettant en oeuvre

(43) Date of publication of application: 30.06.2004
(73) Proprietor: CASTELLINI S.p.A., I-40013 Castel Maggiore (Bologna) (IT)
(72) Inventor: Castellini, Franco, 40124 Bologna (IT)
(74) Representative: Lanzoni, Luciano

(56) References cited:
- EP-A- 0 368 818
- EP-A- 0 734 692
- EP-A- 0 945 140
- EP-A- 1 344 499
- US-A- 4 545 956
- US-A- 5 785 523
- US-B1- 6 286 527

## Description

The present invention relates to a method for sterilising / disinfecting the water circuit of a dental unit and to a dental unit implementing the method.

In the manufacturing of dental units, one of the fundamental parts, if not the "heart", of a dental unit is its water and air circuit, where the water line supplies fluids used by dental equipment and patients (water or physiological saline for tumblers and handpieces), or consumer units (swilling water for the spittoon), while the air line is used for certain items of equipment (air spray handpieces, cooling air and driving air).

With increases in general standards of hygiene and with dental apparatus and equipment becoming more and more "fragile", several design solutions have been found for the water and air circuits of dental units, not only to guarantee their efficient operation and durability but also to maintain the sterility of the conduits during patient treatments.

Considering that the basic structure of these fluid circuits comprises a first main line supplying water from the mains, and a second main line supplying air from an external source (compressor), each of which has a plurality of branches leading to the operating and accessory equipment of the dental unit, different systems have been designed on the basis of different methods aimed at improving the functioning and disinfection of these fluid lines or parts of them.

In particular, the present specification focuses attention on the water line which is disinfected according to two different methods, one with a continuous cycle and the other with a discontinuous cycle, and both requiring additional devices to be fitted to the basic structure of the circuit.

The present specification is concerned in particular with the solutions based on the discontinuous disinfection/sterilisation cycle. In this type of cycle, as disclosed in patent publications EP - 111.249 and EP - 317.521 (the latter being by the present Applicant), the mains water supply is shut off, and a "dedicated" branch equipped with an independent tank is used to feed sterilising liquid into the conduits that supply water to the handpieces.

After a preset time, depending on the quality of disinfection/sterilisation required and the properties of the sterilising liquid, the line is opened again and the sterilising liquid drained out.

The drainage of the sterilising liquid is performed by flushing water (or a user fluid) supplied by the main line (or by a dedicated line) and opening the control valves on the handpieces so that the water or user fluid expels the sterilising liquid, rinses the water line and flows out into an appropriate drain.

Although this method, which has been used on dental units for some time, has proved to be very effective and practical, the Applicant, has found, after in-depth research conducted in accordance with its policy of continual improvement of dental unit sanitising procedures, that the sanitising product or products used require a considerable amount of time to take action.

The aim of the present invention is to overcome the above mentioned drawback by providing a sterilising / disinfecting method, and a dental unit that implements the method, that is practical and fast and, at the same, gives excellent results in sanitising the water circuit of the dental unit.

The above mentioned aim is achieved in a method for sterilising / disinfecting the water circuit of a dental unit as described in claim 1.

The technical characteristics of the invention, with reference to the above aims, are clearly described in the claims below and its advantages are apparent from the detailed description which follows, with reference to the accompanying drawings which illustrate a preferred embodiment of the invention provided merely by way of example without restricting the scope of the inventive concept, and in which:
- Figure 1 is a diagram of the water circuit of a dental unit implementing the sterilising / disinfecting method according to the present invention;
- Figure 2 is a schematic detail view, with some parts cut away in order to better illustrate others, of the dental unit implementing the sterilising / disinfecting method according to the invention.

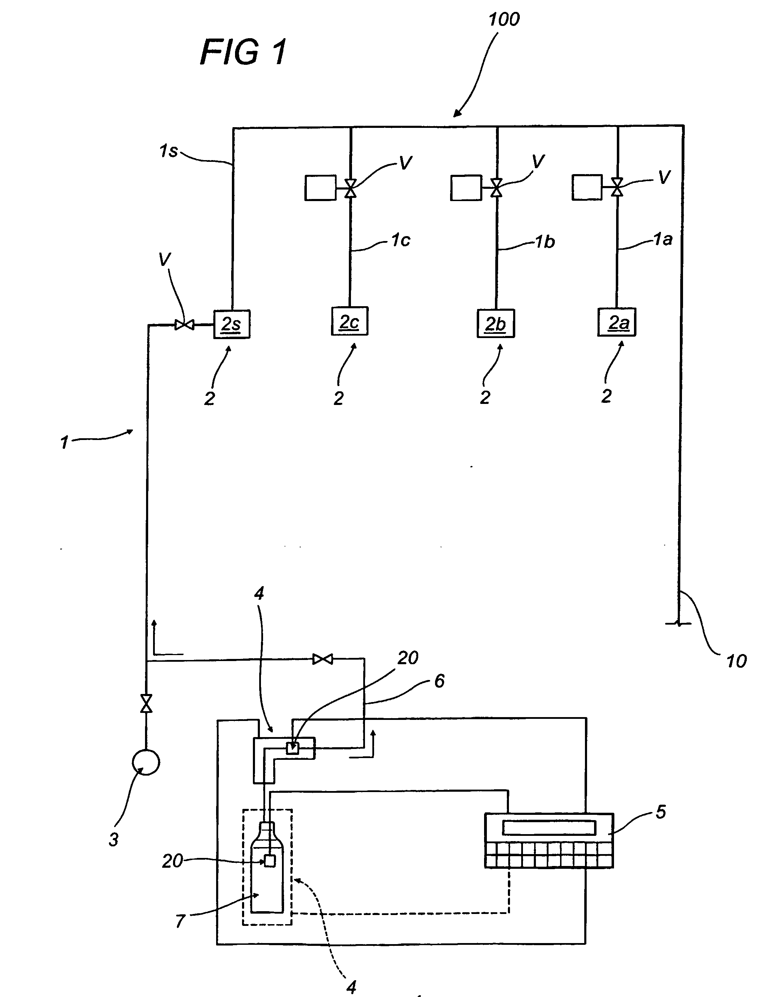

With reference to the accompanying drawings, in particular Figure 1, the method according to the invention is used to sterilise / disinfect the water circuit 1 of a dental unit that is labelled 100 in its entirety.

The dental unit 100 comprises, at least insofar as is relevant to the present invention, medical instruments, such as dental handpieces 2 (for example, a micromotor 2a, a turbine 2b, an ablator 2c and a syringe 2s).

The dental unit 100 also comprises the water circuit 1 that supplies a liquid, which may come from a mains supply 3 or from other containers that are not illustrated, and a drain 10 for the liquid itself.

The water circuit 1 is divided into a plurality of branches 1s, 1a, 1b, 1c, each leading to one of the handpieces 2 present on the dental unit 100, necessitating a fluid supply and equipped with shutoff valves V.

The sterilising / disinfecting method comprises the following steps, at least insofar as is relevant to the present invention:
- introducing a disinfectant / sterilising liquid in the water circuit 1 for a predetermined length of time;
- draining the disinfectant / sterilising liquid out of the circuit 1 through the circuit legs or branches 1a, 1b, 1c, 1s.

Obviously and as is well within the knowledge of experts in the trade familiar with methods of this kind, the shutoff valves V are opened and closed during these steps in order to allow the liquids to flow into the water circuit 1, be held there and then drained out.

In the method according to the invention, the step of introducing the disinfectant / sterilising liquid is accompanied by a step of heating the liquid to a predetermined temperature higher than ambient temperature, using suitable means 4.

More specifically, the disinfectant / sterilising liquid may be heated while the liquid is being introduced in the water circuit 1, that is to say, the step of introducing it and the step of heating it are simultaneous.

Alternatively, the step of heating the disinfectant / sterilising liquid may be carried out just before the step of introducing the liquid, immediately upstream of the water circuit 1 (as described in more detail below).

If the disinfectant / sterilising liquid consists of a first ingredient mixed with a second ingredient to form a third active compound, heating also has the purpose of enhancing the activity of the third active compound produced by the reaction between the two ingredients.

For example, the step of heating the disinfectant / sterilising liquid may bring the liquid up to a temperature of between 30° and 55°, and preferably between 35° and 45°.

Where the dental unit 100 comprises a microprocessor unit 5 that controls its main and auxiliary functions (which are not illustrated since they are of well-known type), the heating step, like all the other steps in the cycle, is controlled by the microprocessor unit 5 acting on the heating means 4. This enables the heating means 4 to be switched on and off in accordance with: the required temperature (which might vary from liquid to liquid); the temperature reached by the liquid (checked during inflow); the point in time the liquid starts flowing into the water circuit 1.

To further improve and speed up the sterilising / disinfecting cycle, the step of introducing the disinfectant / sterilising liquid might be preceded by a step of introducing a gaseous fluid designed to completely expel all the liquid previously inside the water circuit 1.

Preferably, but without restricting the scope of the invention, the step of introducing the heated disinfectant / sterilising liquid in the water circuit 1 may comprise two or more separate sub-steps of introducing defined quantities of the liquid (to fill the circuit 1), alternated with steps of waiting for the next quantity of liquid to be introduced. Further, liquid inflow during each sub-step might be associated with a different temperature from the temperature associated with the sub-steps before and after it, so as to optimise the efficiency and speed of the sterilising / disinfecting cycle: this might also depend on the type of product used. In brief, the purpose is to control the variables that influence the sterilising / disinfecting action in order to optimise this action: the variables involved are hold time, operating temperature and concentration of the disinfectant / sterilising product used.

The dental unit 100 that implements the method described above comprises the water circuit 1 for supplying liquids to each handpiece 2 and at least one first independent branch 6 connected to the water circuit 1 and supplying the water circuit 1 with at least one disinfectant / sterilising liquid from a container 7.

The dental unit 100 comprises means 4 for heating the disinfectant / sterilising liquid, the heating means 4 being mounted on the dental unit 100.

More specifically, the heating means 4 may be mounted between the container 7 and the first branch 6 that supplies the disinfectant / sterilising liquid.

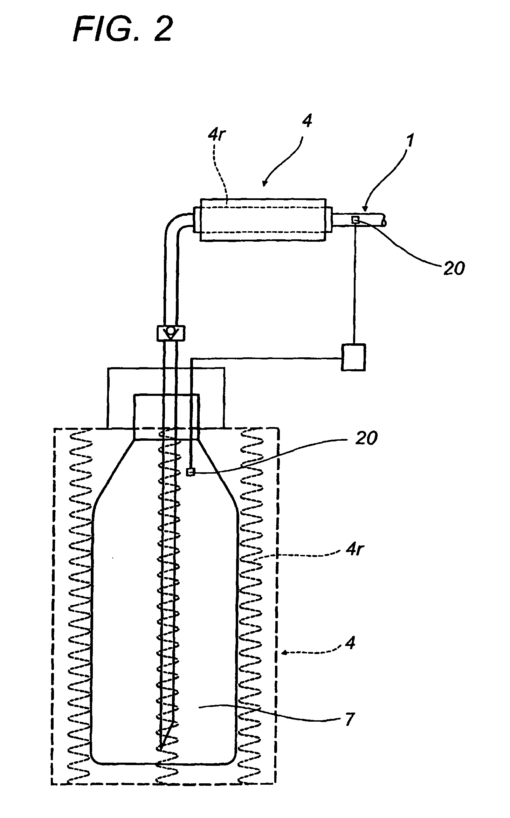

Looking in more detail, with reference to Figure 2, the heating means 4 may be located on the first branch 6 that supplies the disinfectant / sterilising liquid.

The presence on the dental unit 100, of the aforementioned microprocessor unit 5 that controls the main and auxiliary functions of the dental unit 100 itself means that the heating means 4 can be directly controlled by the microprocessor unit 5 (illustrated as a block) which switches the heating means on or off when the disinfectant / sterilising liquid starts flowing into the water circuit 1.

Structurally, the heating means 4 may consist of one or more heating elements 4r wound around the first branch 6 that supplies the disinfectant / sterilising liquid. Alternatively, the heating means 4 may consist of one or more heating elements 4r wound around the container 7 for the liquid in the dental unit 100.

The heating means 4 may also be equipped with sensor means 20 used to check the temperature of the disinfectant / sterilising liquid and connected to the microprocessor unit 5 so as to enable the latter to control the heating means 4 according to the temperature of the liquid.

The method as described above achieves the aforementioned aims by improving and speeding up the sterilising / disinfecting cycle thanks to the simple addition of a heating step which, by raising the temperature of the disinfectant / sterilising liquid, enables the latter to be activated more quickly to sanitise dental unit conduits so that it needs to be held in the water circuit for shorter lengths of time compared to prior art methods.

The dental unit is not encumbered by additional equipment and thus the traditional structure of the dental unit remains practically unchanged.

The invention described can be subject to modifications and variations without thereby departing from the scope of the inventive concept. Moreover, all the details of the invention may be substituted by technically equivalent elements.

## Claims

1. A method for sterilising/disinfecting the water circuit (1) of a dental unit (100) at least comprising medical instruments, such as dental handpieces (2), and a water circuit (1) for supplying a fluid from a main supply (3) through corresponding circuit legs or branches (1a, 1b, 1c, 1s); the method comprising at least the following steps: introducing the disinfectant / sterilising liquid in the water circuit (1) for a predetermined length of time and draining the disinfectant / sterilising liquid out of the circuit (1) through the circuit legs or branches (1a, 1b, 1c, 1s), the method being **characterised in that** the step of introducing the disinfectant / sterilising liquid is accompanied by a step of heating the liquid to a predetermined temperature higher than ambient temperature, using suitable means (4); said heating step being carried out during the step of introducing the disinfectant / sterilising liquid, that is to say, inside the water circuit (1).

2. The method according to claim 1, where the disinfectant / sterilising liquid consists of a first ingredient mixed with a second ingredient, **characterised in that** the step of heating the disinfectant / sterilising liquid is carried out after the first and second ingredients have been mixed.

3. The method according to claim 1, **characterised in that** the step of heating the disinfectant / sterilising liquid brings the liquid to a temperature of between 30° and 55°.

4. The method according to claim 1, **characterised in that** the step of heating the disinfectant / sterilising liquid brings the liquid to a temperature of between 35° and 45°.

5. The method according to claim 1, where the dental unit comprises a microprocessor unit (5) to control the main and auxiliary functions of the dental unit (100), **characterised in that** the heating step is controlled by the microprocessor unit (5) acting on the heating means (4) so that the heating means (4) are switched on and off in accordance with the temperature reached by the liquid and the point in time the liquid starts flowing into the water circuit (1).

6. The method according to claim 1, **characterised in that** the step of introducing the disinfectant / sterilising liquid in the water circuit (1) is preceded by a step of introducing a gaseous fluid so that all the user liquid previously inside the water circuit (1) is expelled.

7. The method according to claim 6, **characterised in that** the step of introducing the gaseous fluid includes a step of heating the gaseous fluid to the same temperature as that of the disinfectant / sterilising liquid.

8. The method according to claim 1, **characterised in that** the step of introducing the disinfectant / sterilising liquid in the water circuit (1) comprises two or more sub-steps of introducing defined quantities of the liquid for defined lengths of time, alternated with steps of waiting for the next quantity of liquid to be introduced.

9. The method according to claim 8, **characterised in that** each sub-step of introducing the disinfectant / sterilising liquid in the water circuit (1) comprises a heating step to keep the liquid at the same temperature as in the previous or the following sub-step.

10. The method according to claim 8, **characterised in that** each sub-step of introducing the disinfectant / sterilising liquid in the water circuit (1) comprises a step of heating the liquid to a different temperature from that of the previous or the following sub-step.

11. A dental unit comprising at least one main water circuit (1) for supplying a liquid to each handpiece (2) or accessory item of dental equipment present on the dental unit (100), and at least one independent circuit branch (6) connected to the water circuit (1) and supplying the water circuit (1) with at least one disinfectant / sterilising liquid from a container (7), the dental unit being **characterised in that** it comprises means (4) for heating the disinfectant / sterilising liquid and mounted on the dental unit (100); the heating means (4) being positioned between the container (7) and the first branch (6) that supplies the disinfectant / sterilising liquid.

12. The dental unit according to claim 11, including a microprocessor unit (5) to control the main and auxiliary functions of the dental unit (100), **characterised in that** the heating means (4) are controlled by the microprocessor unit (5) which switches the heating means (4) on or off when the disinfectant / sterilising liquid starts flowing into the water circuit (1).

13. The dental unit according to claim 11, **characterised in that** the heating means (4) consist of at least one heating element (4r) wound around the first branch (6) that supplies the disinfectant / sterilising liquid.

14. The dental unit according to claim 11, **characterised in that** the heating means (4) consist of at least one heating element (4r) wound around the container (7) for the disinfectant / sterilising liquid in the dental unit (100)

15. The dental unit according to claim 12, **characterised in that** the heating means (4) are equipped with sensor means (20) connected to the microprocessor unit (5) and used to check the temperature of the disinfectant / sterilising liquid.

## Patentansprüche

1. Vorrichtung zur Sterilisation/Desinfektion des Wasserkreislaufs (1) einer zahnärztlichen Einheit (100), welche wenigsten medizinische Instrumente enthält, wie zahnärztliche Handstücke (2), sowie einen Wasserkreislauf (1) zum Zuführen einer Flüssigkeit von einem Hauptnetz (3) über entsprechende Zweigleitungen oder Abzweigungen (1a, 1b, 1c, 1s); wobei das Verfahren wenigstens die folgenden Phasen enthält: Eingeben der Desinfizier-/Sterilisierflüssigkeit in den Wasserkreislauf (1) für eine bestimmte Dauer und Ablassen der Desinfizier-/Sterilisierflüssigkeit aus dem Wasserkreislauf (1) durch die Zweigleitungen oder Abzweigungen (1a, 1b, 1c, 1s), wobei das Verfahren **dadurch gekennzeichnet ist, dass** die Phase des Eingebens der Desinfizier-/Sterilisierflüssigkeit durch eine Phase des Erwärmens der Flüssigkeit unter Verwendung von geeigneten Mitteln (4) auf eine Temperatur begleitet wird, die höher ist als die Raumtemperatur; wobei die genannte Erwärmungsphase während der Phase des Eingebens der Desinfizier-/Sterilisierflüssigkeit durchgeführt wird, das heisst im Inneren des Wasserkreislaufs (1).

2. Verfahren nach Patentanspruch 1, bei welchem die Desinfizier-/Sterilisierflüssigkeit aus einem ersten Ingredienz besteht, das mit einem zweiten Ingredienz gemischt ist, **dadurch gekennzeichnet, dass** die Phase des Erwärmens der Desinfizier-/Sterilisierflüssigkeit durchgeführt wird, nachdem die ersten und zweiten Ingredienzien gemischt worden sind.

3. Verfahren nach Patentanspruch 1, **dadurch gekennzeichnet, dass** die Phase des Erwärmens der Desinfizier-/Sterilisierflüssigkeit die Flüssigkeit auf eine Temperatur zwischen 30° und 55° bringt.

4. Verfahren nach Patentanspruch 1, **dadurch gekennzeichnet, dass** die Phase des Erwärmens der Desinfizier-/Sterilisierflüssigkeit die Flüssigkeit auf eine Temperatur zwischen 35° und 45° bringt.

5. Verfahren nach Patentanspruch 1, bei welchem die zahnärztliche Einheit eine Mikroprozessoreinheit (5) enthält, um die Haupt- und Nebenfunktionen der zahnärztlichen Einheit (100) zu steuern, **dadurch gekennzeichnet, dass** die Erwärmungsphase durch die Mikroprozessoreinheit (5) gesteuert wird, die auf die Heizmittel (4) wirkt, so dass die Heizmittel (4) ein- und abgeschaltet werden, je nach der von der Flüssigkeit erreichten Temperatur und dem Zeitpunkt, zu dem die Flüssigkeit in den Wasserkreislauf (1) zu fliessen beginnt.

6. Verfahren nach Patentanspruch 1, **dadurch gekennzeichnet, dass** der Phase des Eingebens der Desinfizier-/Sterilisierflüssigkeit in den Wasserkreislauf (1) eine Phase des Einführens eines gasförmigen Fluids vorausgeht, so dass alle vorher in dem Wasserkreislauf (1) vorhandene Benutzerflüssigkeit entfernt wird.

7. Verfahren nach Patentanspruch 6, **dadurch gekennzeichnet, dass** die Phase des Eingebens des gasförmigen Fluids eine Phase des Erwärmens des gasförmigen Fluids enthält, und zwar bis auf die gleiche Temperatur wie die der Desinfizier-/Sterilisierflüssigkeit.

8. Verfahren nach Patentanspruch 1, **dadurch gekennzeichnet, dass** die Phase des Eingebens der Desinfizier-/Sterilisierflüssigkeit in den Wasserkreislauf (1) zwei oder mehrere Unterphasen des Eingebens bestimmter Mengen der Flüssigkeit für eine festgelegte Dauer enthält, die sich mit Phasen des Wartens auf die nächste einzugebende Flüssigkeitsmenge abwechseln.

9. Verfahren nach Patentanspruch 8, **dadurch gekennzeichnet, dass** jede Unterphase des Eingebens der Desinfizier-/Sterilisierflüssigkeit in den Wasserkreislauf (1) eine Erwärmungsphase enthält, um die Flüssigkeit auf derselben Temperatur zu halten wie in der vorangegangenen oder der nachfolgenden Phase.

10. Verfahren nach Patentanspruch 8, **dadurch gekennzeichnet, dass** jede Unterphase des Eingebens der Desinfizier-/Sterilisierflüssigkeit in den Wasserkreislauf (1) eine Erwärmungsphase der Flüssigkeit auf eine andere Temperatur enthält, als die der vorangegangenen oder der nachfolgenden Phase.

11. Zahnärztliche Einheit, enthaltend wenigstens einen Hauptwasserkreislauf (1) zum Zuführen einer Flüssigkeit an jedes Handstück (2) oder Zubehör der zahnärztlichen Ausrüstung, die an der zahnärztlichen Einheit (100) vorhanden ist, und wenigstens eine unabhängige Zweigleitung (6), angeschlossen an den Wasserkreislauf (1) und den Wasserkreislauf (1) mit wenigstens einer Desinfizier-/Sterilisierflüssigkeit aus einem Behälter (7) speisend, wobei die zahnärztliche Einheit **dadurch gekennzeichnet ist, dass** sie Mittel (4) zum Erwärmen der Desinfizier-/Sterilisierflüssigkeit enthält, montiert an der zahnärztlichen Einheit (100); und wobei die Heizmittel (4) zwischen dem Behälter (7) und der ersten, die Desinfizier-/Sterilisierflüssigkeit zuführenden Zweigleitung (6) positioniert sind.

12. Zahnärztliche Einheit nach Patentanspruch 11, enthaltend eine Mikroprozessoreinheit (5) zum Steuern der Haupt-und Nebenfunktionen der zahnärztlichen Einheit (100), **dadurch gekennzeichnet, dass** die Heizmittel (4) durch die Mikroprozessoreinheit (5) gesteuert werden, welche die Heizmittel (4) ein- und abschaltet, wenn die Desinfizier-/Sterilisierflüssigkeit beginnt, in den Wasserkreislauf (1) zu fliessen.

13. Zahnärztliche Einheit nach Patentanspruch 11, **dadurch gekennzeichnet, dass** die Heizmittel (4) aus wenigstens einem Heizelement (4r) bestehen, das um die erste Zweigleitung (6) gewunden ist, welche die Desinfizier-/Sterilisierflüssigkeit zuführt.

14. Zahnärztliche Einheit nach Patentanspruch 11, **dadurch gekennzeichnet, dass** die Heizmittel (4) aus wenigstens einem Heizelement (4r) bestehen, das um den Behälter (7) für die Desinfizier-/Sterilisierflüssigkeit in der zahnärztlichen Einheit (100) gewunden ist.

15. Zahnärztliche Einheit nach Patentanspruch 12, **dadurch gekennzeichnet, dass** die Heizmittel (4) mit Fühlermitteln (20) ausgestattet sind, angeschlossen an die Mikroprozessoreinheit (5) und dazu benutzt, die Temperatur der Desinfizier-/Sterilisierflüssigkeit zu erfassen.

## Revendications

1. Une méthode pour stériliser / désinfecter le circuit d'eau (1) d'une unité pour soins dentaires (100) comprenant au moins des instruments médicaux, tels que des pièces à main dentaires (2), et un circuit d'eau (1) pour alimenter un fluide provenant d'une alimentation principale (3) à travers des branches ou ramifications de circuit (1a, 1b, 1c, 1s) ; la méthode comprenant au moins les phases suivantes : introduction du liquide de désinfection / stérilisation dans le circuit d'eau (1) pendant un intervalle de temps prédéterminé et évacuation du liquide de désinfection / stérilisation hors du circuit (1) à travers les branches ou ramifications (1a, 1b, 1c, 1s), la méthode étant **caractérisée en ce que** la phase d'introduction du liquide de désinfection / stérilisation est accompagnée d'une phase de chauffage du liquide à une température prédéterminée supérieure à la température ambiante, en utilisant des moyens (4) prévus à cet effet ; ladite phase de chauffage étant effectuée durant la phase d'introduction du liquide de désinfection / stérilisation, c'est-à-dire à l'intérieur du circuit d'eau (1).

2. La méthode selon la revendication 1, où le liquide de désinfection / stérilisation est composé d'un premier ingrédient mélangé à un second ingrédient, **caractérisée en ce que** la phase de chauffage du liquide de désinfection / stérilisation est effectuée une fois que les premier et second ingrédients ont été mélangés.

3. La méthode selon la revendication 1, **caractérisée en ce que** la phase de chauffage du liquide de désinfection / stérilisation porte le liquide à une température comprise entre 30° et 55°.

4. La méthode selon la revendication 1, **caractérisée en ce que** la phase de chauffage du liquide de désinfection / stérilisation porte le liquide à une température comprise entre 35° et 45°.

5. La méthode selon la revendication 1, où l'unité pour soins dentaires comprend une unité à microprocesseur (5) destinée à commander les fonctions principales et auxiliaires de ladite unité pour soins dentaires (100), **caractérisée en ce que** la phase de chauffage est commandée par l'unité à microprocesseur (5) qui agit sur les moyens de chauffage (4) afin que ces mêmes moyens de chauffage (4) soient activés et désactivés en fonction de la température atteinte par le liquide et du moment où le liquide se met à affluer dans le circuit d'eau (1).

6. La méthode selon la revendication 1, **caractérisée en ce que** la phase d'introduction du liquide de désinfection / stérilisation dans le circuit d'eau (1) est précédée d'une phase d'introduction d'un fluide gazeux à même de chasser tout le liquide d'utilisation déjà présent à l'intérieur du circuit d'eau (1).

7. La méthode selon la revendication 6, **caractérisée en ce que** la phase d'introduction du fluide gazeux inclut une phase de chauffage du fluide gazeux à la même température que celle du liquide de désinfection / stérilisation.

8. La méthode selon la revendication 1, **caractérisée en ce que** la phase d'introduction du liquide de désinfection / stérilisation dans le circuit d'eau (1) comprend deux sous-phases ou plus consistant à introduire des quantités définies du liquide pendant des intervalles de temps donnés, alternées avec des phases d'attente de la quantité suivante de liquide à introduire.

9. La méthode selon la revendication 8, **caractérisée en ce que** chaque sous-phase d'introduction du liquide de désinfection / stérilisation dans le circuit d'eau (1) comprend une phase de chauffage afin de maintenir le liquide à la même température que lors de la sous-phase précédente ou suivante.

10. La méthode selon la revendication 8, **caractérisée en ce que** chaque sous-phase d'introduction du liquide de désinfection / stérilisation dans le circuit d'eau (1) comprend une phase de chauffage du liquide à une température différente de celle de la sous-phase précédente ou suivante.

11. Une unité pour soins dentaires comprenant au moins un circuit d'eau principal (1) pour alimenter un liquide à chaque pièce à main (2) ou accessoire dentaire présent sur ladite unité pour soins dentaires (100), et au moins une branche de circuit indépendante (6) reliée au circuit d'eau (1) et alimentant ce même circuit d'eau (1) avec au moins un liquide de désinfection / stérilisation provenant d'un récipient (7), l'unité pour soins dentaires étant **caractérisée en ce qu'**elle comprend des moyens (4) destinés à chauffer le liquide de désinfection / stérilisation et montés sur l'unité pour soins dentaires (100) ; les moyens de chauffage (4) étant positionnés entre le récipient (7) et la première branche (6) qui alimente le liquide de désinfection / stérilisation.

12. L'unité pour soins dentaires selon la revendication 11, comprenant une unité à microprocesseur (5) à même de commander les fonctions principales et auxiliaires de ladite unité pour soins dentaires (100), **caractérisée en ce que** les moyens de chauffage (4) sont commandés par l'unité à microprocesseur (5) qui active ou désactive ces mêmes moyens de chauffage (4) quand le liquide de désinfection / stérilisation se met à affluer dans le circuit d'eau (1).

13. L'unité pour soins dentaires selon la revendication 11, **caractérisée en ce que** les moyens de chauffage (4) consistent en au moins un élément chauffant (4r) enroulé autour de la première branche (6) qui alimente le liquide de désinfection / stérilisation.

14. L'unité pour soins dentaires selon la revendication 11, **caractérisée en ce que** les moyens de chauffage (4) consistent en au moins un élément chauffant (4r) enroulé autour du récipient (7) contenant le liquide de désinfection / stérilisation situé dans l'unité pour soins dentaires (100).

15. L'unité pour soins dentaires selon la revendication 12, **caractérisée en ce que** les moyens de chauffage (4) sont équipés de moyens capteurs (20) reliés à l'unité à microprocesseur (5) et utilisés pour vérifier la température du liquide de désinfection / stérilisation.
